# EUROPÄISCHE PATENTSCHRIFT

(11) **EP 1 395 187 B1**
(45) Veröffentlichungstag und Bekanntmachung des Hinweises auf die Patenterteilung: **18.08.2010**
(21) Anmeldenummer: 02754581.3
(22) Anmeldetag: 23.05.2002
(51) Int. Cl.: A61B 17/68

(54) **IMPLANTAT ZUR FIXIERUNG BENACHBARTER KNOCHENPLATTEN**
IMPLANT FOR FIXING ADJACENT OSSEOUS PLATES
IMPLANT POUR FIXER DES PLAQUES OSSEUSES VOISINES

(30) Priorität: 15.06.2001 DE 10128918
(43) Veröffentlichungstag der Anmeldung: 10.03.2004
(73) Patentinhaber: Aesculap AG, 78532 Tuttlingen (DE)
(72) Erfinder: NESPER, Markus, 78532 Tuttlingen (DE); STEINHILPER, Klaus-Dieter, 78532 Tuttlingen (DE); WEISSHAUPT, Dieter, 78194 Immendingen (DE); LERCH, Karl-Dieter, 58452 Witten (DE)
(74) Vertreter: Regelmann, Thomas
(86) Internationale Anmeldenummer: PCT/EP2002/005652
(87) Internationale Veröffentlichungsnummer: WO 2002/102258

(56) Entgegenhaltungen:
- DE-C- 19 952 359
- US-A- 6 068 631
- US-B1- 6 238 395

## Beschreibung

Die Erfindung betrifft ein Implantat zur Fixierung benachbarter Knochenplatten, insbesondere Schädelknochenplatten, mit einem inneren Anlageelement, durch welches ein Trennspalt zwischen den Knochenplatten auf einer Knochenplatteninnenseite überdeckbar ist und mit einem äußeren Anlageelement zur Überdeckung des Trennspalts auf einer der Knochenplatteninnenseite gegenüberliegenden Knochenplattenaußenseite.

Bei Gehirnoperationen muss, um Zugriff zu dem zu operierenden Gehirngewebe zu erhalten, die Schädeldecke geöffnet werden. Dazu werden eine oder mehrere Knochenplatten aus dem Schädel herausgesägt. Mit Abschluss der Gehirnoperation müssen diese Knochenplatten wieder eingesetzt werden und an den Knochenplatten des restlichen Schädels fixiert werden. Dazu sind entsprechende Implantate vorgesehen, welche im Körper des Patienten verbleiben.

Aus der DE 199 52 359 C1 ist ein chirurgisches Verbindungselement zur Fixierung benachbart angeordneter Knochenplatten bekannt, welches ein erstes Anlageelement, ein zweites Anlageelement und ein Kopplungselement, mittels welchem erstes und zweites Anlageelement miteinander koppelbar sind, so dass zwischen dem ersten und zweiten Anlageelement liegende Knochenplatten fixierbar sind, umfasst. Das Kopplungselement kann ein Faden oder Draht sein.

Der Erfindung liegt die Aufgabe zugrunde, ein Implantat zur Fixierung benachbarter Knochenplatten der eingangs genannten Art zu schaffen, welches durch den Operateur einfach zu bedienen ist und mit dem sich die Knochenplatten sicher fixieren lassen.

Diese Aufgabe wird erfindungsgemäß dadurch gelöst, dass mindestens ein Spannband, dessen Breite größer als seine Höhe ist, durch das äußere Anlageelement verschieblich geführt ist und mit dem bei Ausübung einer Zugspannung das innere Anlageelement und das äußere Anlageelement miteinander verspannbar sind, und dass das mindestens eine Spannband an dem äußeren Anlageelement fixierbar ist, wobei ein oder mehrere Hakenelemente zur Fixierung des mindestens einen Spannbands vorgesehen sind, und das mindestens eine Spannband bezüglich des äußeren Anlageelements einhakbar ist.

Durch das Festziehen eines Spannbands lassen sich das innere Anlageelement und das äußere Anlageelement aufeinander zu bewegen und miteinander verspannen, so dass die benachbarten Knochenplatten miteinander fixiert sind. Diese Spannstellung wiederum lässt sich sichern, indem das Spannband an dem äußeren Anlageelement fixiert wird. Die Verspannung lässt sich dabei von dem Operateur auf einfache Weise durchführen, ohne dass ein zusätzliches Applikationswerkzeug benötigt wird.

Ein Spannband lässt sich von dem Operateur auf einfache Weise handhaben, da es eine genügend große Breite zum Anfassen und zum Ausüben einer Zugspannung aufweist.

Ein Spannband stellt darüber hinaus eine genügende Fläche bereit, um eine Fixierung an dem äußeren Anlageelement bewirken zu können. Insbesondere sind zur Sicherung der Fixierungsstellung der Knochenplatten Hakenelemente vorgesehen, welche das Spannband durchdringen können und somit diese Fixierungsstellung sichern.

Darüber hinaus lässt sich das erfindungsgemäße Implantat auch auf einfache Weise herstellen, da das innere Anlageelement und das äußere Anlageelement getrennt von dem Spannband herstellbar sind und sich die Verbindung zwischen Spannband und den Anlageelementen auf einfache Weise durch Durchfädeln herstellen lässt.

Darüber hinaus lässt sich das erfindungsgemäße Implantat auch auf vorteilhafte Weise einsetzen: Mittels des Spannbands kann beim Einsetzen einer Knochenplatte in eine Schädelausnehmung das innere Anlageelement gehalten werden, bis die gewünschte relative Positionierung der Knochenplatten erreicht ist. Es lässt sich dann danach das äußere Anlageelement über das Spannband führen und so die Verspannung durchführen.

Ein Spannband lässt sich dabei je nach Anwendung aus einem resorbierbaren oder nicht-resorbierbaren Material herstellen. Als resorbierbare Materialien sind insbesondere synthetische Materialien oder organische Materialien einsetzbar. Als nicht-resorbierbare Materialien sind Kunststoffe wie PEEK oder auch metallische Materialien wie Titan oder andere biologisch verträgliche Werkstoffe einsetzbar.

Die Breite des mindestens einen Spannbands ist größer als seine Höhe und insbesondere wesentlich größer ist, beispielsweise mindestens fünffach größer. Dadurch wird eine entsprechende Fläche bereitgestellt, die das Anfassen für einen Operateur erleichtert und die eine einfache Fixierung des Spannbands an dem äußeren Anlageelement mittels Hakenelementen erlaubt.

Die Fixierung der Knochenplatten lässt sich auf einfache Weise sichern, wenn das mindestens eine Spannband bezüglich des äußeren Anlageelements einhakbar ist. Das Spannband stellt eine große Fläche zum Eingriff und insbesondere zum Durchdringen von Hakenelementen bereit, um so das Spannband bezüglich des äußeren Anlageelements fixieren zu können.

Um weiterhin die Handhabung zu erleichtern und eine sichere Fixierung an dem äußeren Anlageelement zu erhalten, liegt dabei die Breite des mindestens einen Spannbands im Bereich zwischen 25 % und 75 % einer Breitenabmessung eines Anlageelements. Insbesondere liegt dabei die Breite im Bereich von ca. der Hälfte dieser Breitenabmessung, die bei einem runden, scheibenförmigen Anlageelement der Durchmesser ist.

Insbesondere ist es vorgesehen, daß das mindestens eine Spannband biegeflexibel ausgebildet ist, um so ein Heranziehen der beiden Anlageelemente relativ zueinander und damit eine Verspannung relativ zueinander bewirken zu können.

Es ist vorgesehen, daß das mindestens eine Spannband an dem inneren Anlageelement gehalten ist, um so bei der Verspannung einen Festpunkt bereitzustellen, d. h. sicherzustellen, daß sich das Spannband nicht mehr bezüglich des inneren Anlageelements bewegt.

Es kann dabei grundsätzlich vorgesehen sein, daß das mindestens eine Spannband an dem inneren Anlageelement festgelegt ist, d. h. an diesem bezüglich seines Haltepunkts unveränderlich fixiert ist.

Ganz besonders vorteilhaft ist es aber, wenn ein Spannband durch das innere Anlageelement durchgeführt ist und dabei das Spannband an dem inneren Anlageelement mittels eines Spannbandbogens gehalten ist. Dadurch läßt sich das Spannband an dem inneren Anlageelement fixieren, wobei jedoch diese Fixierung veränderlich ist. Dadurch wird zum einen die Herstellung erleichtert, und zum anderen ist aufgrund des Haltens mittels eines Spannbandbogens eine große Sicherheit erreicht, daß sich das innere Anlageelement und das Spannband nicht voneinander lösen.

Fertigungstechnisch günstig ist es, wenn das innere Anlageelement zwei beabstandete Öffnungen zur Durchführung des Spannbands aufweist. Zwischen den Öffnungen ist dann eine Art von Steg gebildet, welcher den Spannbandbogen hält, d. h. eine Anlagefläche für diesen bildet.

Vorteilhafterweise sind dabei die Öffnungen so angeordnet und ausgebildet, d. h. das Stegelement auch entsprechend so angeordnet und ausgebildet, daß ein erster Spannbandbereich und ein zweiter Spannbandbereich, zwischen welchen ein Spannbandbogen gebildet ist, bei der Durchsetzung des Trennspalts im wesentlichen parallel zueinander ausrichtbar sind. Dadurch wird verhindert, daß die Spannbandbögen eventuell Querkräfte auf die Knochenplatten ausüben und bei Ausüben einer Zugspannung auf das mindestens eine Spannband eventuell die Knochenplatten gegeneinander verrücken.

Um eine gleichmäßige Anlagefläche für die benachbarten Knochenplatten bereitzustellen, sind vorteilhafterweise die Öffnungen im wesentlichen spiegelsymmetrisch zu einem Zentrum des inneren Anlageelements angeordnet.

Es ist dabei günstig, wenn der Abstand der Öffnungen kleiner als ca. ein Achtel einer Breitenabmessung des inneren Anlageelements ist. Bei einem kreisförmigen Anlageelement ist dabei diese Breitenabmessung der Durchmesser. Auf diese Weise läßt sich sicherstellen, daß der Trennspalt, durch den das Spannband geführt werden muß, nicht zu groß wird und zum anderen läßt sich eine parallele Führung von entsprechenden Spannbandbereichen erreichen. Vorteilhafterweise sind dabei Kanten der Öffnungen abgerundet, um eine Beschädigung des Spannbands zu vermeiden.

Weiterhin ist es günstig, wenn das äußere Anlageelement eine oder mehrere Öffnungen aufweist, durch die jeweils ein Längsende eines Spannbands durchführbar ist. Dadurch läßt sich das äußere Anlageelement mittels des mindestens einen Spannbands gegenüber dem inneren Anlageelement fixieren, und dadurch wiederum lassen sich benachbarte Knochenplatten zwischen den beiden Anlageelementen fixieren.

Um durch ein Festziehen des Spannbands eine Verspannung der beiden Anlageelemente durchführen zu können und dazu das äußere Anlageelement gegenüber dem inneren Anlageelement bewegen zu können, weist eine Öffnung eine Umlenkkante zur Umlenkung eines Spannbands auf, so daß auf das Spannband eine Zugkraft quer zu einer Abstandsrichtung zwischen innerem Anlageelement und äußerem Anlageelement ausübbar ist. Insbesondere ist dabei die Umlenkkante abgerundet, um eine gute Führung des Spannbands in der Öffnung zu gewährleisten und um zum anderen eine Beschädigung des Spannbands zu vermeiden.

Vorteilhafterweise sind dabei die Öffnung oder Öffnungen so angeordnet und ausgebildet, daß das mindestens eine Spannband im wesentlichen senkrecht zu dem Anlageelement im Trennspalt positioniert ist, um so das Ausüben von Querkräften vom Spannband auf die Knochenplatten zu verhindern und die Größe des Trennspalts gering halten zu können.

Eine Fixierung der Knochenplatten zwischen den Anlageelementen lässt sich auf einfache Weise erreichen, wenn auf ein erstes Spannbandende eine Zugkraft mit einer Querkomponente in einer ersten Richtung ausübbar ist und auf ein zweites Spannbandende mit einer Querkomponente in eine Gegenrichtung ausübbar ist. Durch relatives Auseinanderziehen der beiden Spannbandenden wird dann das äußere Anlageelement in Richtung des inneren Anlageelements hin verschoben und eine Verspannung und damit Fixierung der Knochenplatten zwischen den beiden Anlageelementen bewirkt.

Ein erfindungsgemäßes Implantat lässt sich auf einfache Weise herstellen, wenn das erste Spannbandende und das zweite Spannbandende an dem gleichen Spannband gebildet sind, d. h. wenn das Spannband durch das innere Anlageelement durchgeschleift ist und dann die jeweiligen Enden in entgegengerichtete Richtungen geführt sind.

Insbesondere ist es dabei vorteilhaft, wenn ein Hakenelement eine schiefe Flanke und eine steile Flanke aufweist, wobei die steile Flanke einem Zugende des mindestens einen Spannbands zugewandt angeordnet ist. Die steile Flanke ist dabei insbesondere so ausgebildet, dass bei eingehaktem Spannband diese im Wesentlichen senkrecht zu diesem ist. Ein Hakenelement weist dann einen im Wesentlichen dreieckförmigen Querschnitt auf, wobei die steile Flanke diejenige ist, welche bezüglich einer Höhenrichtung des Dreiecks einen steileren Winkel aufweist. Bei der entsprechenden Anordnung der steilen Flanke dem Zugende hin wird gewährleistet, daß beim Einhaken die Spannung nicht vermindert wird, da die Verhakungsfläche eines Hakenelements in einem Bereich des Spannbands einhakt, welches dem inneren Anlageelement, an dem das Spannband gehalten ist, näher liegt und damit bei der Durchführung des Einhakungsvorgangs die Spannkraft erhalten bleibt. Zum anderen ist dadurch aber auch sichergestellt, daß sich die Kräfte auf das Spannband nicht vergrößern, so daß beispielsweise eine vom Operateur gewünschte Positionierung der Knochenplatten relativ zueinander durch vergrößerte Kräfte beim Einhaken nicht zerstört wird.

Bei einer Variante einer Ausführungsform sind das oder die Hakenelemente an dem äußeren Anlageelement angeordnet. Ein Operateur kann dann die Fixierungsstellung der Knochenplatten auf einfache Weise durch Einhaken des Spannbands an dem äußeren Anlageelement durchführen. Insbesondere ist dabei eine Reihe von beabstandeten Hakenelementen vorgesehen, um über eine große Fläche eine Fixierung zu bewirken.

Es kann dabei vorgesehen sein, daß das oder die Hakenelemente auf einer äußeren Oberfläche an dem äußeren Anlageelement angeordnet sind. Insbesondere weisen dabei Hakenspitzen von einer äußeren Oberfläche des äußeren Anlageelements weg.

Es kann alternativ oder auch zusätzlich vorgesehen sein, daß das oder die Hakenelemente in einer Öffnung zur Durchführung des mindestens einen Spannbands angeordnet sind. Bei dieser Variante wird die Fixierung des Spannbands in der Öffnung bewirkt. Hakenspitzen sind dann quer zu einer Abstandsrichtung zwischen innerem Anlageelement und äußerem Anlageelement orientiert.

Bei einer weiteren Ausführungsform ist eine Fixierungskappe zum Aufsetzen auf dem äußeren Anlageelement vorgesehen, wobei das Spannband zwischen dem äußeren Anlageelement und der Fixierungskappe fixierbar ist. Es läßt sich dann die Fixierung der Knochenplatten bewirken und durch Aufsetzen der Fixierungskappe wird diese Stellung geschert.

Ganz besonders vorteilhaft ist es dabei, wenn die Fixierungskappe ein Stegelement aufweist, welches in den Trennspalt einsetzbar ist. Dieses Stegelement bewirkt dann eine zusätzliche Fixierung in dem Trennspalt und zum anderen läßt sich mit dem Stegelement der Trennspalt ausfüllen.

Günstigerweise ist das Stegelement zwischen gegenüberliegenden Spannbandbereichen in den Trennspalt einsetzbar, so daß dadurch eine zusätzliche Sicherung der Spannstellung des Spannbands erreicht ist.

Bei einer Variante einer Ausführungsform sind an dem Stegelement Querlaschen gebildet, welche quer zur Abstandsrichtung zwischen innerem Anlageelement und äußerem Anlageelement elastisch beweglich sind. Dadurch läßt sich bei eingesetztem Stegelement, wenn dies entsprechend an das äußere Anlageelement angepaßt wird, eine elastische Kraft von dem Stegelement auf das Anlageelement ausüben, welches eine zusätzliche Fixierung der Fixierungskappe an dem äußeren Anlageelement bewirkt, wodurch sich wiederum eine gute Sicherung der Fixierung der Knochenplatten erreichen läßt.

Um das Spannband zwischen dem äußeren Anlageelement und der Fixierungskappe zu fixieren, ist die Fixierungskappe bzw. das äußere Anlageelement mit einem oder mehreren Hakenelement versehen und das äußere Anlageelement bzw. die Fixierungskappe mit korrespondierenden Öffnungen zur Aufnahme des oder der Hakenelemente. Dadurch läßt es sich gewährleisten, daß die Hakenelemente das Spannband durchdringen und dieses damit sicher halten. Die Öffnungen sorgen dann dafür, daß die bezüglich des Spannbands überstehenden Hakenelemente aufgenommen werden. Zum anderen kann durch das Eintauchen der Hakenelementen in die Öffnungen eine zusätzliche Fixierung erreicht werden.

Die nachfolgende Beschreibung bevorzugter Ausführungsformen dient im Zusammenhang mit der Zeichnung der näheren Erläuterung der Erfindung. Es zeigen:
- Figur 1: eine seitliche Schnittansicht eines ersten Ausführungsbeispiels eines erfindungsgemäßen Implantats zur Fixierung benach- barter Knochenplatten, wobei eine Fixierungsstellung gezeigt ist;
- Figur 2: eine Schnittansicht in Richtung A auf das Implantat gemäß Figur 1;
- Figur 3: eine Variante des Ausführungsbeispiels gemäß Figur 1 und 2 in einer Schnittansicht in der Richtung A;
- Figur 4: eine Schnittansicht eines zweiten Ausführungsbeispiels eines erfindungsgemäßen Implantats zur Fixierung benachbarter Knochenplatten;
- Figur 5: eine Schnittansicht eines dritten Ausführungsbeispiels eines erfindungsgemäßen Implantats zur Fixierung benachbarter Knochenplatten und
- Figur 6: eine Draufsicht in Richtung B auf das Implantat gemäß Figur 5.

Mittels eines erfindungsgemäßen Implantats lassen sich benachbarte Knochenplatten 10, 12 fixieren. Beispielsweise wird bei Gehirnoperationen die Schädeldecke durch Herausnahme eines oder mehrerer Knochenstücke geöffnet. Nach Beendigung der Operation werden die entsprechenden Knochenstücke wieder eingesetzt, wozu dann die benachbarten Knochenplatten 10 und 12 relativ zueinander positioniert und in einer entsprechenden Endstellung fixiert werden müssen. Zwischen den Knochenplatten 10 und 12 ist dabei ein Trennspalt 14 gebildet, durch den sich Verbindungsmittel 16 zur Verbindung zwischen einem inneren Anlageelement 18 und einem äußeren Anlageelement 20 führen lassen. Sind beispielsweise die Knochenplatten 10 und 12 Schädelknochenplatten, dann wird dabei das innere Anlageelement 18 auf Knochenplatteninnenseiten 22 positioniert, welche dem Schädelinneren zugewandt sind und das äußere Anlageelement 20 wird auf einer gegenüberliegenden Knochenplattenaußenseite 24 positioniert.

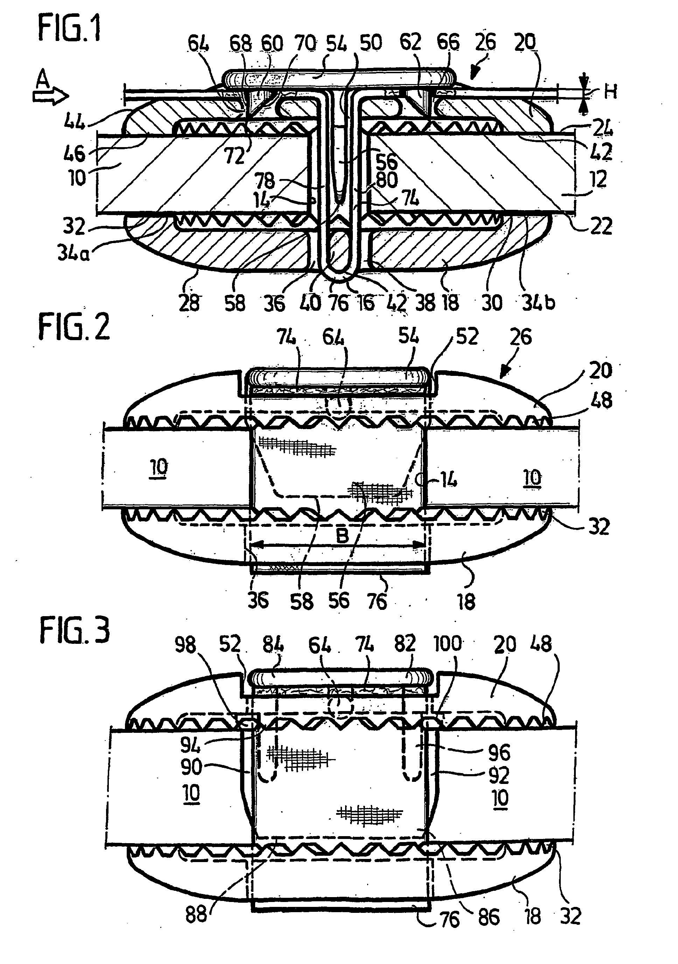

Bei dem in Figur 1 gezeigten und dort als Ganzes mit 26 bezeichneten ersten Ausführungsbeispiel eines erfindungsgemäßen Implantats zur Fixierung benachbarter Knochenplatten 10, 12 ist das innere Anlageelement 18 scheibenförmig ausgebildet, wobei es insbesondere einen kreisförmigen Querschnitt aufweist. Es ist aus einem körperverträglichen Kunststoff, einem resorbierbaren Kunststoff oder aus einem Metall hergestellt. Eine äußere Oberfläche 28 des inneren Anlageelements ist konvex gewölbt. Eine dieser äußeren Oberfläche 28 gegenüberliegende Oberfläche 30 ist mit einem Zahnkranzring 32 versehen, welcher zum besseren Angriff an den Knochenplatten 10 und 12 dient.

Mittels des inneren Anlageelements 18 ist der Trennspalt 14 überdeckbar, so daß der Zahnkranzring 32 in einem Teilbereich 34a an der Knochenplatte 10 anliegt und in einem insbesondere gegenüberliegenden Teilbereich 34b an der Knochenplatte 12 anliegt.

Die Oberfläche 30 ist außerhalb des Zahnkranzrings 32 gegenüber Zahnspitzen zurückgesetzt.

In dem inneren Anlageelement 18 sind in einem zentralen Bereich zwei beabstandete durchgehende schlitzförmige Öffnungen 36, 38 gebildet, welche zwischen den Oberflächen 30 und 28 verlaufen. Getrennt sind sie durch ein Stegelement 40. Dieses Stegelement 40 weist insbesondere zu der äußeren Oberfläche 28 zugewandt abgerundete Kanten 42 auf.

Das äußere Anlageelement 20 ist ebenfalls scheibenförmig ausgebildet und weist insbesondere einen kreisförmigen Querschnitt auf, wobei die äußeren Abmessungen des äußeren Anlageelements 20 im wesentlichen denen des inneren Anlageelements 18 entsprechen. Es ist wie das innere Anlageelement 18 aus einem körperverträglichen Kunststoff, resorbierbaren Kunststoff oder aus Metall hergestellt. Es weist ebenfalls eine konvexe äußere Oberfläche 44 auf und eine gegenüberliegende Oberfläche 46, welche mit einem Zahnkranzring 48 entsprechend dem Zahnkranzring 32 des inneren Anlageelements 18 versehen ist.

In dem inneren Anlageelement ist in einem zentrale Bereich eine durchgehende schlitzförmige Öffnung 50 gebildet, durch die die Verbindungsmittel 16 führbar sind.

In der äußeren Oberfläche 44 ist anschließend an die Öffnung 50 eine quaderförmige Ausnehmung 52 vorgesehen, welche zur Aufnahme einer Fixierungskappe 54 dient. An der Fixierungskappe 54 ist dabei in einem zentralen Bereich und insbesondere einstückig ein Stegelement 56 angeordnet, welches an seinem vorderen Ende 58 keilförmig ausgebildet ist. Dieses Stegelement 56 läßt sich in den Trennspalt 14 zwischen den Knochenplatten 10 und 12 einsetzen und diese so mindestens teilweise ausfüllen und es lassen sich, wie unten noch näher beschrieben, die Verbindungsmittel 16 bezüglich des Trennspalts 14 fixieren.

Die Fixierungskappe 54 weist symmetrisch zu dem Stegelement 56 gegenüberliegende Hakenelemente 60, 62 auf, die in korrespondierende durchgehende Öffnungen 64, 66 des äußeren Anlageelements 20 eintauchen können. Ein Hakenelement 60, 62 weist dabei eine steile Flanke 68 und eine schiefe Flanke 70 auf, so daß ein Hakenelement 60, 62 dreieckförmig ausgebildet ist. Eine Spitze 72 eines Hakenelements 60, 62 weist dabei, wenn die Fixierungskappe 54 in die Ausnehmung 52 eingesetzt ist, in Richtung von der äußeren Oberfläche 44 zur Oberfläche 46, wobei die schiefe Flanke 70 dem zentralen Bereich des äußeren Anlageelements 20 zugewandt ist, d. h. insbesondere dem Stegelement 56 zugewandt ist, während die steile Flanke 68 diesem zentralen Bereich abgewandt angeordnet ist.

Die Verbindungsmittel 16 sind durch ein Spannband 74 gebildet, welches biegeelastisch ist. Dieses Spannband kann aus einem resorbierbaren oder nicht-resorbierbaren Material hergestellt sein. An dem inneren Anlageelement 18 ist es in den Öffnungen 36, 38 an dem Stegelement 40 durch einen Spannbandbogen 76 gehalten, an den sich jeweils ein erster Spannbandbereich 78 und ein zweiter Spannbandbereich 80 anschließen, welche den Trennspalt 14 durchsetzen.

An der Öffnung 50, welche dazu insbesondere abgerundete Kanten aufweist, wird der erste Spannbandbereich 78 umgelenkt und verläuft im wesentlichen parallel zu der äußeren Oberfläche 44 des äußeren Anlageelements 20. Entsprechend wird der zweite Spannbandbereich 80 ebenfalls umgelenkt und verläuft ebenfalls parallel zur äußeren Oberfläche 44 des äußeren Anlageelements 20. Entsprechende Enden des Spannbands 74, welche dem ersten Spannbandbereich 78 und dem zweiten Spannbandbereich 80 zugeordnet sind, liegen dabei abgewandt voneinander und das Stegelement 56 liegt zwischen diesen Enden.

In dem Trennspalt 14 sind die beiden Spannbandbereiche 78 und 80 im Wesentlichen parallel zueinander ausgerichtet.

Das Spannband 74 wird relativ zu dem inneren Anlageelement 18 und dem äußeren Anlageelement 20 dadurch fixiert, dass die Hakenelemente 60, 62 in das Spannband 74 einhaken und diese Hakenelemente 60, 62 dann weiter in die zugeordneten Öffnungen 64, 66 des äußeren Anlageelements 20 eintauchen. Dadurch lässt sich eine Fixierung benachbarter Knochenplatten 10, 12 durch das Implantat 26 erreichen.

Das erfindungsgemäße Implantat 26 funktioniert wie folgt:

Das Spannband 74 ist mittels der durchgehenden Öffnungen 36 und 38 durch das innere Anlageelement 18 geschleift. Es weist dabei eine Breite B auf, welche erheblich größer ist als dessen Höhe H. Es ist weiterhin durch das äußere Anlageelement 20 durch dessen Öffnung 50 geschleift, wobei dann ein Operateur die jeweiligen Enden des Spannbands 74 ergreifen kann.

Die Knochenplatten 10 und 12 und die Anlageelemente 18 und 20 werden so relativ zueinander positioniert, dass zum einen das Spannband 74 mit seinem ersten Spannbandbereich 78 und seinem zweiten Spannbandbereich 80 den Trennspalt 14 durchsetzt, das innere Anlageelement 18 an der Knochenplatteninnenseite 22 anliegt und den Trennspalt 14 überdeckt und das äußere Anlageelement 20 an der Knochenplattenaußenseite 24 anliegt und den Trennspalt 14 ebenfalls überdeckt. Ein Operateur übt dann eine Zugspannung auf jeweils die beiden Enden des Spannbands 74 aus, welche jeweils den Spannbandbereichen 78 und 80 zugeordnet sind, oder hält ein Ende fest und übt eine Zugspannung auf das andere Ende aus. Insbesondere wird dazu das Spannband 74 jeweils umgelegt und parallel zu dem äußeren Anlageelement 20 geführt, so dass der Operateur Zugkräfte auf die jeweiligen Enden ausüben kann, welche bei gegenüberliegenden Enden entgegengesetzt sind. Durch ein derartiges Festziehen des Bandes werden die Anlageelemente 18 und 20 gegeneinander gezogen, so dass eine sichere Fixierung der Knochenplatten 10 und 12 zwischen den Anlageelementen 18 und 20 erreicht ist. Es wird dann anschließend die Fixierungskappe 54 in die Ausnehmung 52 eingesetzt, wobei das Stegelement 56 in den Trennspalt 14 eintaucht. Dadurch wird das Spannband 74 in dem Trennspalt 14 fixiert und auch der Trennspalt 14 durch das Stegelement 56 mindestens teilweise ausgefüllt.

Die Hakenelemente 60, 62 greifen dabei beim Aufsetzen der Fixierungskappe 54 in das Spannband 74 ein und verhaken sich mit diesem. Die Hakenelemente 60 und 62 tauchen in die zugeordneten Öffnungen 64 und 66 ein. Dadurch wird das Spannband 74 mit dem äußeren Anlageelement 20 fixiert und damit wiederum wird die Fixierungsstellung der Knochenplatten 10 und 12 zwischen dem äußeren Anlageelement 20 und dem inneren Anlageelement 18 gesichert.

Die Hakenelemente 60, 62 durchdringen das Spannband 74, d. h. die steile Flanke 68 weist eine Höhe auf, die größer ist als die Höhe H des Spannbands 74. Die Ausbildung eines Hakenelements 60, 62 mit der Anordnung der schiefen Flanke 70 der Öffnung 50 zu und der steilen Flanke 68 dem Ende des Spannbands 74 zu gewährleistet dabei, dass sich eine Verhakung unter Sicherung der Spannstellung ausbildet.

Die Breite B des Spannbands liegt im Bereich zwischen ca. 25 % und 75 % des Durchmessers des äußeren Anlageelements 20 bzw. inneren Anlageelements 18. Dadurch lässt sich zum einen das Spannband von einem Operateur leicht greifen. Zum anderen ist eine große Fläche für Verhakungen mittels der Hakenelemente 60 und 62 bereitgestellt.

Es kann auch vorgesehen sein, dass anstatt eines Hakenelements 60 bzw. 62 eine Reihe von Hakenelementen vorgesehen ist. Es kann weiterhin alternativ vorgesehen sein, dass die Hakenelemente an dem äußeren Anlageelement 20 gebildet sind und die Fixierungskappe 54 dann korrespondierende Öffnungen aufweist.

Eine Variante des ersten Ausführungsbeispiels 26, welche in Figur 3 gezeigt ist, unterscheidet sich im Wesentlichen durch eine andere Ausbildung einer Fixierungskappe 82. Im Übrigen ist dann das entsprechende Implantat gleich ausgebildet wie oben beschrieben und funktioniert gleich. Gleiche Elemente des Implantats gemäß Figur 3 tragen daher die gleichen Bezugsnummern wie beim Implantat 26.

Die Fixierungskappe 82 weist eine runde Deckelscheibe 84 auf, an der einstückig ein Stegelement 86 gebildet ist. An seinem vorderen Ende 88 ist dieses Stegelement keilförmig ausgebildet.

An dem Stegelement 86 sind gegenüberliegende Querlaschen 90, 92 durch jeweilige schlitzförmige Ausnehmungen 94, 96 gebildet, welche im Wesentlichen senkrecht zu der Deckelscheibe 84 verlaufen. An der Deckelscheibe 84 zugewandten Enden sind die Querlaschen 90, 92 mit Halteköpfen 98, 100 versehen.

Durch die Ausnehmungen 94 bzw. 96 ist die Querlasche 90 bzw. 92 quer zur Flächennormale der Deckelscheibe 84 beweglich, d. h. im Bereich der Querlaschen 90, 92 läßt sich die Breite des Stegelements 86 verringern. Diese Verringerung der Breite erfordert einen Kraftaufwand zur elastischen Verformung des Stegelements 86. Dadurch läßt sich das Stegelement 86 durch die Öffnung 50 hindurch in den Trennspalt 14 einschieben, wobei aber die Querlaschen 90, 92 in Richtung der Zentrumsachse der Deckelscheibe 84 elastisch gebogen sind. Bei aufgesetzter und in dem Spannband 74 eingehakter Deckelscheibe 84 üben dabei wiederum die Querlaschen 90, 92 in der Öffnung 50 eine Kraft auf das äußere Anlageelement 20 aus, wobei die Halteköpfe 98, 100 für ein sicheres Halten sorgen. Dadurch wird das Stegelement 86 und damit die Fixierungskappe 82 zusätzlich, neben dem Eintauchen der Hakenelemente 60, 62 in die zugeordneten Öffnungen 64, 66, mit dem äußeren Anlageelement 20 verspannt.

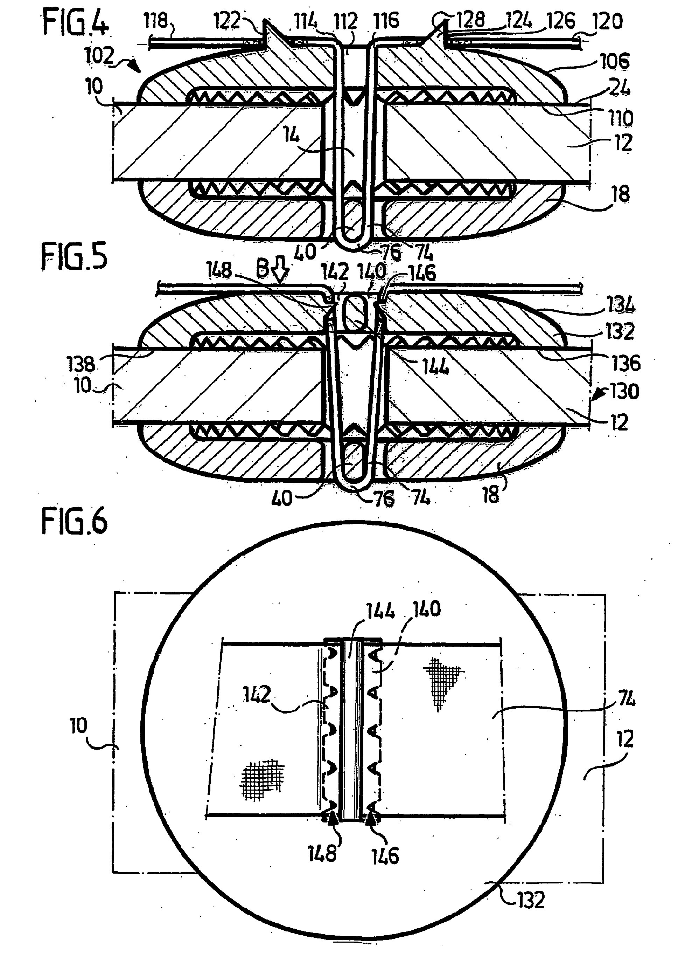

Bei einem zweiten Ausführungsbeispiel, welches in Figur 4 als Ganzes mit 102 bezeichnet ist, ist das innere Anlageelement 18 grundsätzlich gleich ausgebildet wie oben beschrieben. Es werden deshalb gleiche Bezugszeichen verwendet.

Bei dem Implantat 102 ist ein äußeres Anlageelement 104 vorgesehen, welches eine konvexe äußere Oberfläche 106 aufweist und eine dieser abgewandten Oberfläche 108 zur Anlage an eine Knochenplattenaußenseite 24. Diese Oberfläche 108 ist mit einem Zahnkranzring 110 versehen.

Das äußere Anlageelement 104 weist in einem zentralen Bereich eine durchgehende schlitzförmige Öffnung 112 auf, durch die das Spannband 74 führbar ist. Durch die Öffnung 112 sind Umlenkkanten 114, 116 gebildet, welche insbesondere abgerundet sind, um die Richtung des Spannbands von im wesentlichen parallel zu dem Trennspalt 14 in quer zu diesem umzulenken. Durch Ausübung einer Zugspannung auf die jeweiligen Enden 118 und 120 des Spannbands 74 bzw. Festhalten eines Endes und Ausüben einer Zugspannung auf das andere Ende, d. h. durch Festziehen des Spannbands 74 werden das innere Anlageelement 18 und das äußere Anlageelement 104 aufeinander zu gezogen und die dazwischenliegenden Knochenplatten 10 und 12 lassen sich sicher fixieren.

Das äußere Anlageelement 104 weist an seiner äußeren Oberfläche 106 insbesondere symmetrisch zu der Öffnung 112 angeordnet Hakenelemente 122, 124 auf, welche dem jeweiligen Ende 118, 120 des Spannbands 74 zugeordnet sind. Eine steile Flanke 126 eines Hakenelements 122 bzw. 124 weist dabei von der Öffnung 112 weg, während eine schiefe Flanke 128 dieser zugewandt ist.

Nach Festziehen des Spannbands läßt sich dieses dann in die jeweiligen Hakenelemente 122, 124 einhaken, um so die Fixierung der Knochenplatten 10 und 12 mittels der Anlageelemente 18 und 104 zu sichern.

Bei einem dritten Ausführungsbeispiel, welches in Figur 5 und 6 als Ganzes mit 130 bezeichnet ist, ist wiederum das innere Anlageelement 18 grundsätzlich gleich ausgebildet wie oben beschrieben. Ein äußeres Anlageelement 132 weist eine konvexe äußere Oberfläche 134 auf. Dieser abgewandt ist eine Oberfläche 136 des äußeren Anlageelements 132 mit einem Zahnkranzring 138 wie oben beschrieben versehen.

In einem zentralen Bereich sind in dem äußeren Anlageelement 132 zwei beabstandete, durchgehende schlitzförmige Öffnungen 140 und 142 gebildet, welche durch ein Stegelement 144 getrennt sind. Durch diese Öffnungen 140 und 142 ist das Spannband 74 geführt.

In den Öffnungen 140 und 142 ist an dem äußeren Anlageelement 132 jeweils dem Stegelement 144 gegenüberliegend eine Reihe von Hakenelementen 146, 148 gebildet. Ein Haken 150 einer solchen Reihe weist dabei eine steile Flanke auf, welche der äußeren Oberfläche 134 des äußeren Anlageelements 132 zugewandt ist und eine schiefe Flanke, welche der Oberfläche 136 zugewandt angeordnet ist.

Durch die Öffnungen 140 und 142 sind dabei jeweilige Enden des Spannbands 74 gefädelt, und durch Festziehen des Spannbands lassen sich das innere Anlageelement 18 und das äußere Anlageelement 132 gegeneinander ziehen und sich so die Knochenplatten 10 und 12 relativ zueinander durch die Anlageelemente 18 und 132 fixieren. Durch Einhaken des Spannbands in die Haken 150 der Hakenreihen 146 und 148 läßt sich diese Fixierungsstellung sichern.

Bei der erfindungsgemäßen Verwendung eines Spannbands ist kein Applikationswerkzeug zur Fixierung der Knochenplatten 10, 12 zwischen innerem und äußerem Anlageelement notwendig: Ein Operateur kann durch Festziehen des Spannbands 74 die Anlageelemente gegeneinander ziehen und so eine sichere Fixierung der Knochenplatten 10 und 12 relativ zueinander erreichen. Durch Einhaken des Spannbands 74, wobei die entsprechenden Hakenelemente die Struktur des Spannbands 74 durchdringen, wird dann diese Fixierungsstellung gesichert.

Das Spannband 74 kann dabei je nach Anwendung aus einem resorbierbaren Material oder einem nicht-resorbierbaren Material hergestellt sein.

## Patentansprüche

1. Implantat zur Fixierung benachbarter Knochenplatten (10, 12), insbesondere Schädelknochenplatten, mit einem inneren Anlageelement (18), durch welches ein Trennspalt (14) zwischen den Knochenplatten (10, 12) auf einer Knochenplatteninnenseite (22) überdeckbar ist und mit einem äußeren Anlageelement (20; 104; 132) zur Überdeckung des Trennspalts (14) auf einer der Knochenplatteninnenseite (22) gegenüberliegenden Knochenplattenaußenseite (24),
**dadurch gekennzeichnet, dass** mindestens ein Spannband (74), dessen Breite (B) größer als seine Höhe (H) ist, durch das äußere Anlageelement (20; 104; 132) verschieblich geführt ist und mit dem bei Ausübung einer Zugspannung das innere Anlageelement (18) und das äußere Anlageelement (20; 104; 132) miteinander verspannbar sind, und dass das mindestens eine Spannband (74) an dem äußeren Anlageelement (20; 104; 132) fixierbar ist, wobei ein oder mehrere Hakenelementen (60, 62; 122, 124; 150) zur Fixierung des mindestens einen Spannbands (74) vorgesehen sind, und das mindestens eine Spannband (74) bezüglich des äußeren Anlageelements (20; 104; 132) einhakbar ist.

2. Implantat nach Anspruch 1, **dadurch gekennzeichnet, dass** die Breite (B) des mindestens einen Spannbands (74) im Bereich zwischen 25 % und 75 % einer Breitenabmessung eines Anlageelements (18) liegt.

3. Implantat nach einem der vorangehenden Ansprüche, **dadurch gekennzeichnet, dass** das mindestens eine Spannband (74) biegeflexibel ausgebildet ist.

4. Implantat nach einem der vorangehenden Ansprüche, **dadurch gekennzeichnet, dass** das mindestens eine Spannband (74) an dem inneren Anlageelement (18) gehalten ist.

5. Implantat nach Anspruch 4, **dadurch gekennzeichnet, dass** das mindestens eine Spannband an dem inneren Anlageelement festgelegt ist.

6. Implantat nach Anspruch 4, **dadurch gekennzeichnet, dass** ein Spannband (74) durch das innere Anlageelement (18) durchgeführt ist.

7. Implantat nach Anspruch 6, **dadurch gekennzeichnet, dass** das Spannband (74) an dem inneren Anlageelement (18) mittels eines Spannbandbogens (76) gehalten ist.

8. Implantat nach Anspruch 6 oder 7, **dadurch gekennzeichnet, dass** das innere Anlageelement (18) zwei beabstandete Öffnungen (36, 38) zur Durchführung des Spannbands (74) aufweist.

9. Implantat nach Anspruch 8, **dadurch gekennzeichnet, dass** die Öffnungen (36, 38) so angeordnet und ausgebildet sind, dass ein erster Spannbandbereich (78) und ein zweiter Spannbandbereich (80), zwischen welchen ein Spannbandbogen (76) gebildet ist, bei der Durchsetzung des Trennspalts (14) im wesentlichen parallel zueinander ausrichtbar sind.

10. Implantat nach Anspruch 8 oder 9, **dadurch gekennzeichnet, dass** die Öffnungen (36, 38) im wesentlichen spiegelsymmetrisch zu einem Zentrum des inneren Anlageelements (18) angeordnet sind.

11. Implantat nach einem der Ansprüche 8 bis 10, **dadurch gekennzeichnet, dass** der Abstand der Öffnungen (36, 38) kleiner als ein Achtel einer Breitenabmessung des inneren Anlageelements (18) ist.

12. Implantat nach einem der Ansprüche 8 bis 11, **dadurch gekennzeichnet, dass** Kanten der Öffnungen (36, 38) abgerundet sind.

13. Implantat nach einem der vorangehenden Ansprüche, **dadurch gekennzeichnet, dass** das äußere Anlageelement (20; 104; 132) eine oder mehrere Öffnungen (50; 112; 140, 142) aufweist, durch die jeweils ein Längsende eines Spannbands (74) durchführbar ist.

14. Implantat nach Anspruch 13, **dadurch gekennzeichnet, dass** eine Öffnung (50; 112; 140, 142) eine Umlenkkante zur Umlenkung eines Spannbands (74) aufweist, so dass auf das Spannband eine Zugkraft quer zu einer Abstandsrichtung zwischen innerem Anlageelement (18) und äußerem Anlageelement (20; 104; 132) ausübbar ist.

15. Implantat nach Anspruch 14, **dadurch gekennzeichnet, dass** die Umlenkkante abgerundet ist.

16. Implantat nach einem der Ansprüche 13 bis 15, **dadurch gekennzeichnet, dass** die Öffnung (50; 112) oder Öffnungen (140, 142) so angeordnet und ausgebildet sind, dass das mindestens eine Spannband im wesentlichen senkrecht zu den Anlageelementen (18, 20; 18, 104; 18, 132) im Trennspalt (14) positioniert ist.

17. Implantat nach einem der vorangehenden Ansprüche, **dadurch gekennzeichnet, dass** auf ein erstes Spannbandende eine Zugkraft mit einer Querkomponente in einer ersten Richtung ausübbar ist und auf ein zweites Spannbandende eine Zugkraft mit einer Querkomponente in eine Gegenrichtung ausübbar ist.

18. Implantat nach Anspruch 17, **dadurch gekennzeichnet, dass** das erste Spannbandende und das zweite Spannbandende an dem gleichen Spannband (74) gebildet sind.

19. Implantat nach einem der vorangehenden Ansprüche, **dadurch gekennzeichnet, dass** ein Hakenelement (60) eine schiefe Flanke (70) und eine steile Flanke (68) aufweist, wobei die steile Flanke (68) einem Zugende des mindestens einen Spannbands (74) zugewandt angeordnet ist.

20. Implantat nach einem der vorangehenden Ansprüche, **dadurch gekennzeichnet, dass** das oder die Hakenelemente (122, 124; 150) an dem äußeren Anlageelement (104; 132) angeordnet sind.

21. Implantat nach Anspruch 20, **dadurch gekennzeichnet, dass** eine Reihe (146, 148) von beabstandeten Hakenelementen (150) vorgesehen ist.

22. Implantat nach Anspruch 20 oder 21, **dadurch gekennzeichnet, dass** das oder die Hakenelemente (122, 124) auf einer äußeren Oberfläche (106) an dem äußeren Anlageelement (104) angeordnet sind.

23. Implantat nach Anspruch 22, **dadurch gekennzeichnet, dass** Hakenspitzen von einer äußeren Oberfläche (106) des äußeren Anlageelements (104) weg weisen.

24. Implantat nach Anspruch 20 oder 21, **dadurch gekennzeichnet, dass** das oder die Hakenelemente (150) in einer Öffnung (140, 142) zur Durchführung des mindestens einen Spannbands (74) angeordnet sind.

25. Implantat nach Anspruch 24, **dadurch gekennzeichnet, dass** Hakenspitzen quer zu einer Abstandsrichtung zwischen innerem Anlageelement (18) und äußerem Anlageelement (132) orientiert sind.

26. Implantat nach einem der vorangehenden Ansprüche, **dadurch gekennzeichnet, dass** eine Fixierungskappe (54; 82) zum Aufsetzen auf das äußere Anlageelement (20) vorgesehen ist, wobei das Spannband (74) zwischen dem äußeren Anlageelement (20) und der Fixierungskappe (54; 82) fixierbar ist.

27. Implantat nach Anspruch 26, **dadurch gekennzeichnet, dass** die Fixierungskappe (54; 82) ein Stegelement (56; 86) aufweist, welches in den Trennspalt (14) einsetzbar ist.

28. Implantat nach Anspruch 27, **dadurch gekennzeichnet, dass** das Stegelement (56; 86) zwischen gegenüberliegenden Spannbandbereichen (78, 80) in den Trennspalt (14) einsetzbar ist.

29. Implantat nach Anspruch 27 oder 28, **dadurch gekennzeichnet, dass** an dem Stegelement (86) Querlaschen (90, 92) gebildet sind, welche quer zur Abstandsrichtung zwischen innerem Anlageelement (18) und äußerem Anlageelement (20) elastisch beweglich bezüglich des äußeren Anlageelements (20) sind.

30. Implantat nach einem der Ansprüche 26 bis 29, **dadurch gekennzeichnet, dass** die Fixierungskappe (54; 82) bzw. das äußere Anlageelement (20) mit einem oder mehreren Hakenelementen (60, 62) versehen ist und das äußere Anlageelement (20) bzw. die Fixierungskappe (54; 82) mit korrespondierenden Öffnungen (64, 66) zur Aufnahme des oder der Hakenelemente (60, 62).

## Claims

1. Implant for fixing adjacent bone plates (10, 12), in particular cranial bone plates, comprising an inner abutment element (18) by means of which a separation gap (14) between the bone plates (10, 12) can be overlapped at a bone plate inner side (22) and comprising an outer abutment element (20; 104; 132) for overlapping the separation gap (14) at a bone plate outer side (24) lying opposite the bone plate inner side (22),
**characterized in that** extending displaceably through the outer abutment element (20; 104; 132) is at least one tension band (74), the width (B) of which is greater than its height (H) and by means of which, when tension is exerted, the inner abutment element (18) and the outer abutment element (20; 104; 132) are mutually braceable, and that the at least one tension band (74) is fixable on the outer abutment element (20; 104; 132), wherein one or more hook elements (60, 62; 122, 124; 150) are provided for fixing the at least one tension band (74) and the at least one tension band (74) can be hooked in relative to the outer abutment element (20; 104; 132).

2. Implant according to claim 1, **characterized in that** the width (B) of the at least one tension band (74) is in the region of between 25% and 75% of a width dimension of an abutment element (18).

3. Implant according to one of the preceding claims, **characterized in that** the at least one tension band (74) is of a flexibly bendable design.

4. Implant according to one of the preceding claims, **characterized in that** the at least one tension band (74) is held on the inner abutment element (18).

5. Implant according to claim 4, **characterized in that** the at least one tension band is fastened to the inner abutment element.

6. Implant according to claim 4, **characterized in that** a tension band (74) is passed through the inner abutment element (18).

7. Implant according to claim 4, **characterized in that** the tension band (74) is held on the inner abutment element (18) by means of a tension band bend (76).

8. Implant according to claim 6 or 7, **characterized in that** the inner abutment element (18) has two spaced-apart openings (36, 38) for passing the tension band (74) through.

9. Implant according to claim 8, **characterized in that** the openings (36, 38) are disposed and designed in such a way that a first tension band region (78) and a second tension band region (80), between which a tension band bend (76) is formed, during penetration of the separation gap (14) are alignable substantially parallel to one another.

10. Implant according to claim 8 or 9, **characterized in that** the openings (36, 38) are disposed substantially mirror-symmetrically relative to a centre of the inner abutment element (18).

11. Implant according to one of claims 8 to 10, **characterized in that** the spacing of the openings (36, 38) is less than an eighth of a width dimension of the inner abutment element (18).

12. Implant according to one of claims 8 to 11, **characterized in that** edges of the openings (36, 38) are rounded off.

13. Implant according to one of the preceding claims, **characterized in that** the outer abutment element (20; 104; 132) has one or more openings (50; 112; 140, 142), through which in each case one longitudinal end of a tension band (74) is passable.

14. Implant according to claim 13, **characterized in that** an opening (50; 112; 140, 142) has a deflection edge for deflecting a tension band (74), so that a tensile force is exertable upon the tension band transversely of a direction of spacing between inner abutment element (18) and outer abutment element (20; 104; 132).

15. Implant according to claim 14, **characterized in that** the deflection edge is rounded off.

16. Implant according to one of claims 13 to 15, **characterized in that** the opening (50; 112) or openings (140, 142) are disposed and designed in such a way that the at least one tension band (74) is positioned substantially at right angles to the abutment elements (18, 20; 18, 104; 18, 132) in the separation gap (14).

17. Implant according to one of the preceding claims, **characterized in that** a tensile force with a transverse component in a first direction is exertable upon a first tension band end and a tensile force with a transverse component in an opposite direction is exertable upon a second tension band end.

18. Implant according to claim 17, **characterized in that** the first tension band end and the second tension band end are formed on the same tension band (74).

19. Implant according to one of the preceding claims, **characterized in that** a hook element (60) has an inclined flank (70) and a steep flank (68), wherein the steep flank (68) is arranged facing a pulling end of the at least one tension band (74).

20. Implant according to one of the preceding claims, **characterized in that** the hook element or elements (122, 124; 150) are disposed on the outer abutment element (104; 132).

21. Implant according to claim 20, **characterized in that** a row (146, 148) of spaced-apart hook elements (150) is provided.

22. Implant according to claim 20 or 21, **characterized in that** the hook element or elements (122, 124) are disposed on an outer surface (106) of the outer abutment element (104).

23. Implant according to claim 22, **characterized in that** hook tips are directed away from an outer surface (106) of the outer abutment element (104).

24. Implant according to claim 20 or 21, **characterized in that** the hook element or elements (150) are disposed in an opening (140, 142) for passing the at least one tension bend (74) through.

25. Implant according to claim 24, **characterized in that** hook tips are orientated transversely of a direction of spacing between inner abutment element (18) and outer abutment element (134).

26. Implant according to one of the preceding claims, **characterized in that** a fixation cap (54; 82) is provided for mounting onto the outer abutment element (20), wherein the tension band (74) is fixable between the outer abutment element (20) and the fixation cap (54; 82).

27. Implant according to claim 26, **characterized in that** the fixation cap (54; 82) comprises a web element (56; 86), which is insertable into the separation gap (14).

28. Implant according to claim 27, **characterized in that** the web element (56; 86) is insertable between opposite-lying tension band regions (78, 80) into the separation gap (14).

29. Implant according to claim 27 or 28, **characterized in that** formed on the web element (86) are transverse tabs (90, 92), which are elastically movable relative to the outer abutment element (20) transversely of the direction of spacing between inner abutment element (18) and outer abutment element (20).

30. Implant according to one of claims 26 to 29, **characterized in that** the fixation cap (54; 82) and/or the outer abutment element (20) is provided with one or more hook elements (60, 62) and the outer abutment element (20) and/or the fixation cap (54; 82) is provided with corresponding openings (64, 66) for receiving the hook element or elements (60, 62).

## Revendications

1. Implant pour fixer ou assembler des plaques osseuses ou os plats (10, 12) voisins, notamment des os plats de boite crânienne, et comprenant un élément d'appui intérieur (18) par lequel peut être recouvert, sur le côté intérieur (22) des os plats, un interstice de sectionnement (14) entre les os plats (10, 12), et comprenant également un élément d'appui extérieur (20; 104; 132) pour recouvrir l'interstice de sectionnement (14) sur un côté extérieur (24) des os plats, qui est opposé au côté intérieur (22) des os plates,
**caractérisé en ce qu'**au moins une bande de serrage (74), dont la largeur (B) est supérieure à sa hauteur ou à son épaisseur (H), est guidée de manière coulissante à travers l'élément d'appui extérieur (20; 104; 132), et permet, en y exerçant une tension de traction, de serrer l'un vers l'autre l'élément d'appui intérieur (18) et l'élément d'appui extérieur (20; 104; 132), et **en ce que** ladite au moins une bande de serrage (74) peut être fixée à l'élément d'appui extérieur (20; 104; 132), un ou plusieurs éléments de crochet (60, 62; 122, 124; 150) étant prévus pour fixer ladite au moins une bande de serrage (74) et ladite au moins une bande de serrage (74) pouvant être accrochée relativement à l'élément d'appui extérieur (20; 104; 132).

2. Implant selon la revendication 1, **caractérisé en ce que** la largeur (B) de ladite au moins une bande de serrage (74) se situe dans une plage comprise entre 25% et 75% d'une dimension de largeur d'un élément d'appui (18).

3. Implant selon l'une des revendications précédentes, **caractérisé en ce que** ladite au moins une bande de serrage (74) est réalisée souple en flexion.

4. Implant selon l'une des revendications précédentes, **caractérisé en ce que** ladite au moins une bande de serrage (74) est maintenue sur l'élément d'appui intérieur (18).

5. Implant selon la revendication 4, **caractérisé en ce que** ladite au moins une bande de serrage est fixée à l'élément d'appui intérieur.

6. Implant selon la revendication 4, **caractérisé en ce qu'**une bande de serrage (74) traverse l'élément d'appui intérieur (18).

7. Implant selon la revendication 6, **caractérisé en ce que** la bande de serrage (74) est maintenue sur l'élément d'appui intérieur (18) au moyen d'une partie courbée (76) de la bande de serrage.

8. Implant selon la revendication 6 ou la revendication 7, **caractérisé en ce que** l'élément d'appui intérieur (18) présente deux ouvertures (36, 38) pour y faire passer la bande de serrage (74).

9. Implant selon la revendication 8, **caractérisé en ce que** les ouvertures (36, 38) sont agencées et réalisées de façon à ce qu'une première zone de bande de serrage (78) et une deuxième zone de bande de serrage (80), entre lesquelles est formée une partie courbée (76) de bande de serrage, puissent être orientées sensiblement de manière parallèle l'une à l'autre au niveau de leur traversée de l'interstice de sectionnement (14).

10. Implant selon la revendication 8 ou la revendication 9, **caractérisé en ce que** les ouvertures (36, 38) sont agencées sensiblement de manière symétrique par rapport à un centre de l'élément d'appui intérieur (18).

11. Implant selon l'une des revendications 8 à 10, **caractérisé en ce que** la distance d'espacement des ouvertures (36, 38) est inférieure à un huitième d'une dimension de largeur de l'élément d'appui intérieur (18).

12. Implant selon l'une des revendications 8 à 11, **caractérisé en ce que** les bords des ouvertures (36, 38) sont arrondis.

13. Implant selon l'une des revendications précédentes, **caractérisé en ce que** l'élément d'appui extérieur (20; 104; 132) présente une ou plusieurs ouvertures (50; 112; 140, 142) à travers lesquelles il est possible de faire passer respectivement une extrémité longitudinale d'une bande de serrage (74).

14. Implant selon la revendication 13, **caractérisé en ce qu'**une ouverture (50; 112; 140, 142) présente un bord de renvoi destiné à dévier une bande de serrage (74), de manière à pouvoir exercer sur la bande de serrage, une force de traction transversalement à une direction d'espacement entre l'élément d'appui intérieur (18) et l'élément d'appui extérieur (20; 104; 132).

15. Implant selon la revendication 14, **caractérisé en ce que** le bord de renvoi est arrondi.

16. Implant selon l'une des revendications 13 à 15, **caractérisé en ce que** l'ouverture (50; 112) ou les ouvertures (140, 142) sont agencées et réalisées de façon à ce que ladite au moins une bande de serrage soit positionnée, dans l'interstice de sectionnement (14), sensiblement de manière perpendiculaire aux éléments d'appui (18, 20; 18, 104; 18, 132).

17. Implant selon l'une des revendications précédentes, **caractérisé en ce que** sur une première extrémité de bande de serrage peut être exercée une force de traction avec une composante transversale dans une première direction, et sur une deuxième extrémité de bande de serrage peut être exercée une force de traction avec une composante transversale dans une direction opposée.

18. Implant selon la revendication 17, **caractérisé en ce que** la première extrémité de bande de serrage et la deuxième extrémité de bande de serrage sont formées sur la même bande de serrage (74).

19. Implant selon l'une des revendications précédentes, **caractérisé en ce qu'**un élément de crochet (60) présente un flanc oblique (70) et un flanc abrupt (68), le flanc abrupt (68) étant agencé de manière à être dirigé vers une extrémité de traction de ladite au moins une bande de serrage (74).

20. Implant selon l'une des revendications précédentes, **caractérisé en ce que** l'élément ou les éléments de crochet (122, 124; 150) sont agencés sur l'élément d'appui extérieur (104; 132).

21. Implant selon la revendication 20, **caractérisé en ce qu'**il est prévu une rangée (146, 148) d'éléments de crochet (150) mutuellement espacés.

22. Implant selon la revendication 20 ou la revendication 21, **caractérisé en ce que** l'élément ou les éléments de crochet (122, 124) sont agencés sur une surface extérieure (106) sur l'élément d'appui extérieur (104).

23. Implant selon la revendication 22, **caractérisé en ce que** des pointes de crochet font saillie d'une surface extérieure (106) de l'élément d'appui extérieur (104).

24. Implant selon la revendication 20 ou la revendication 21, **caractérisé en ce que** l'élément ou les éléments de crochet (150) sont agencés dans une ouverture (140, 142) pour le passage de ladite au moins une bande de serrage (74).

25. Implant selon la revendication 24, **caractérisé en ce que** des pointes de crochet sont orientées transversalement à une direction d'espacement entre l'élément d'appui intérieur (18) et l'élément d'appui extérieur (132).

26. Implant selon l'une des revendications précédentes, **caractérisé en ce qu'**il est prévu un chapeau de fixation (54; 82) destiné à être rapporté sur l'élément d'appui extérieur (20), la bande de serrage (74) pouvant être fixée entre l'élément d'appui extérieur (20) et le chapeau de fixation (54; 82).

27. Implant selon la revendication 26, **caractérisé en ce que** le chapeau de fixation (54; 82) présente un élément de nervure (56; 86), qui peut être inséré dans l'interstice de sectionnement (14).

28. Implant selon la revendication 27, **caractérisé en ce que** l'élément de nervure (56; 86) peut être inséré dans l'interstice de sectionnement (14), entre des zones de bande de serrage (78, 80) en regard l'une de l'autre.

29. Implant selon la revendication 27 ou la revendication 28, **caractérisé en ce que** sur l'élément de nervure (86) sont formées des pattes transversales (90, 92), qui sont mobiles élastiquement par rapport à l'élément d'appui extérieur (20), transversalement à la direction d'espacement entre l'élément d'appui intérieur (18) et l'élément d'appui extérieur (20).

30. Implant selon l'une des revendications 26 à 29, **caractérisé en ce que** le chapeau de fixation (54; 82), respectivement l'élément d'appui extérieur (20), est pourvu d'un ou de plusieurs éléments de crochet (60, 62), et l'élément d'appui extérieur (20), respectivement le chapeau de fixation (54; 82), est pourvu d'ouvertures (64, 66) correspondantes destinées à recevoir l'élément ou les éléments de crochet (60, 62).
